# EUROPÄISCHE PATENTSCHRIFT

(11) **EP 1 250 416 B1**
(45) Veröffentlichungstag und Bekanntmachung des Hinweises auf die Patenterteilung: **31.05.2006**
(21) Anmeldenummer: 01909695.7
(22) Anmeldetag: 26.01.2001
(51) Int. Cl.: C12M 3/00, C12M 1/34, C12N 5/06, C12M 1/22

(54) **Künstlich hergestelltes, dreidimensionales Säugerherzmuskelgewebe und Herstellungsverfahren**
Artificial three-dimensional mammal heart muscle tissue and process for its manufacture
Tissu tridimensionel artificiel de muscle cardiaque de mammifère et procédé de fabrication

(30) Priorität: 27.01.2000 DE 10003521
(43) Veröffentlichungstag der Anmeldung: 23.10.2002
(73) Patentinhaber: Eschenhagen, Prof. Dr., Thomas, 20257 Hamburg (DE)
(72) Erfinder: ESCHENHAGEN, Thomas, 91054 Erlangen (DE); Prof. Dr. Wolfram-Hubertus ZIMMERMANN, D-20253 Hamburg (DE)
(74) Vertreter: Leidescher, Thomas
(86) Internationale Anmeldenummer: PCT/EP2001/000856
(87) Internationale Veröffentlichungsnummer: WO 2001/055297

(56) Entgegenhaltungen:
- EP-A- 0 218 065
- EP-A- 0 751 215
- DE-A- 19 500 498
- DE-A- 19 843 234
- US-A- 3 540 985
- US-A- 3 902 972
- US-A- 3 985 608
- US-A- 4 140 582
- US-A- 4 975 377
- US-A- 5 153 136
- PATENT ABSTRACTS OF JAPAN vol. 014, no. 406 (C-0754), 4. September 1990 (1990-09-04) & JP 02 154679 A (MOCHIDA PHARMACEUT CO LTD), 14. Juni 1990 (1990-06-14)
- PATENT ABSTRACTS OF JAPAN vol. 018, no. 245 (M-1603), 11. Mai 1994 (1994-05-11) & JP 06 032345 A (SONY CORP), 8. Februar 1994 (1994-02-08)

## Beschreibung

Die vorliegende Erfindung betrifft ein künstlich hergestelltes, dreidimensionales Säugerherzmuskelgewebe sowie ein Verfahren zum Kültivieren einer Zellkultür in einer Trägersubstanz.

Die Herzinsuffizienz ist mit einer geschätzten Prävalenz von 1,5 bis 2% der Bevölkerung eine der wichtigsten Ursachen von Morbidität und Mortalität in der westlichen Welt. Die Herzinsuffizienz ist grundsätzlich entweder die Folge einer primär gesteigerten Belastung des Herzens oder die Folge eines Untergangs bzw. einer Schädigung von Herzmuskelzellen (Kardiomyocyten), die, bei gleichbleibendem Bedarf des Organismus an kontraktiler Gesamtleistung des Herzens, sekundär zu einer vermehrten Belastung der verbleibenden, nicht geschädigten Herzmuskelzellen führt.

Dies hat zwei wesentliche Konsequenzen. Einerseits führt die vermehrte Belastung zu einem Größenwachstum, d.h. zu einer Hypertrophie, der verbleibenden, nicht geschädigten Herzmuskelzellen. Andererseits kommt es zu einer Vielzahl von quantitativen Änderungen der Genexpression, d.h. zu einem krankheitsspezifischen Phänotyp der verbleibenden, nicht geschädigten Herzmuskelzellen. Diese spezifischen Änderungen der Genexpression und ihre Folgen für die Funktion der Herzmuskelzellen sind erst zu einem geringen Teil bekannt. Klar ist in diesem Zusammenhang bisher nur, daß sich jede einzelne Zelle (und nicht nur das gesamte Organ Herz) eines insuffizienten Herzens von denen eines gesunden Herzens unterscheidet.

Bisherige therapeutische Bemühungen zur Behandlung der Herzinsuffizienz zielen im wesentlichen darauf ab, das erkrankte Herz hämodynamisch zu entlasten und die Mechanismen, die man als ursächlich für die Veränderungen der Genexpression und der Hypertrophie erkannt hat (Aktivierung von neurohumoralen Systemen wie Sympathikus, Renin-Angiotensin-Aldosteron-System, Endothelin, Wachstumsfaktoren, Entzündungsmediatoren), pharmakologisch zu unterdrücken. Auf diese Weise kann man tatsächlich den natürlichen Verlauf der Erkrankung verzögern. Dabei ist zu berücksichtigen, daß die Aktivierung der vorgenannten Prozesse einerseits sicher sinnvolle Anpassungen darstellen, mit denen ein erkranktes Herz auf die vermehrte Beanspruchung reagiert. Andererseits sind es aber die selben Mechanismen, die letztendlich zu einem vermehrten Untergang von Herzmuskelzellen beitragen und dadurch einen Teufelskreis in Gang setzen, der die schlechte Prognose von Patienten mit Herzinsuffizienz erklärt.

Neue Therapieansätze zur Behandlung der Herzinsuffizienz zielen daher darauf ab, im Herzen spezifische Ziele (Targets) zu identifizieren, die ausschließlich oder überwiegend zu einer verminderten Funktion des Herzens bzw. zu einem beschleunigten Untergang von Herzmuskelzellen führen, und diese Prozesse von solchen Prozessen abzugrenzen, die eher sinnvolle Anpassungen des erkrankten Herzens darstellen. Dazu wurden in den vergangenen Jahren von verschiedenen pharmazeutischen Unternehmen systematische Genscreening-Verfahren eingesetzt, mit denen nach Unterschieden in der Genexpression von insuffizienten und gesunden menschlichen Herzen gesucht wird (subtraktive Verfahren). Mit dem Aufkommen der DNA-Chip-Technologie wird, in Zusammenhang mit dem Humangenomprojekt ("human genome project"), in naher Zukunft die Zahl von neuen, in ihrer Funktion und pathophysiologischen Bedeutung unbekannten, Genen rapide zunehmen.

Es besteht somit ein großer Bedarf nach Methoden, die eine schnelle und effiziente Targetvalidierung erlauben. Das bedeutet, daß man relativ rasch Informationen zu der funktionellen Bedeutung von quantitativen Änderungen der Genexpression unbekannter oder wenig bekannter Gene in Herzmuskelzellen gewinnen möchte und, darüber hinaus gehend, die Wirkung von Substanzen testen, die eine Wirkung auf die Produkte dieser Gene entfalten.

In der deutschen Patentschrift DD 299 439 A5 ist ein Verfahren zur Untersuchung von medizinisch-pharmazeutischen Präparaten an *in vitro* kultivierten Zellverbänden aus Säugetieren, speziell der Maus, beschrieben, bei dem durch Beobachtung die Wirkung dieser Präparate an pulsierenden, differenzierten Herzmuskelzellen untersucht wird. Dazu wird eine Zellinie solcher pluripotenter, embryonaler Stammzellen verwendet, die in spontan pulsierende Herzmuskelzellen differenzieren kann. Aus den embryonalen Stammzellen werden in geeigneter Weise embryoide Körper ("embryoid bodies") kultiviert, die dann dem zu untersuchenden Wirkstoff ausgesetzt werden können, wodurch *in vivo* Untersuchungen nicht mehr nötig sind. Die in der DD 299 439 A5 beschriebene Verfahrensweise erlaubt aber keine Aussagen zum Einfluß der untersuchten Wirkstoffe auf die Kontraktionskraft der Herzmuskelzellen. Es wird lediglich die Pulsänderung mittels einer Stoppuhr untersucht.

In der deutschen Offenlegungsschrift DE 195 00 498 A1 wird zum erstenmal beschrieben, wie man Muskelgewebszellen derart kultivieren kann, so daß daran Messungen der isometrischen Kraftparameter und deren Beeinflussung möglich sind. Dazu sind in der vorgenannten Offenlegungsschrift Matrix- oder Muskelkörper gezeigt, welche zwei plattenförmige, parallel angeordnete, in eine Spann- und Meßvorrichtung einhängbare Halteteile, eine dazwischen gespannte Matrix aus erstarrtem Kollagengel und darin eingebettetes, durch Kultivierung entstandenenes Muskelgewebe aufweisen, wobei die Matrix mit den Halteteilen verbunden ist. Die beiden plattenförmigen Halteteile bestehen aus nichtporösem Material und sind entweder vollmasiv oder hohl. Damit die Halteteile in eine Spann- und Meßvorrichtung eingehängt werden können, sind an diesen röhrchenförmige Elemente ausgebildet. Um zwischen der Matrix und den Halteteilen eine Verbindung mit hoher Zugfestigkeit herzustellen, sind die Oberflächen der Halteteile mit selbstverhakenden Teilen versehen. Des weiteren sind zwischen den Halteteilen Abstandshalter vorgesehen, die während der Herstellung des Matrixkörpers den Abstand der Halteteile voneinander konstant halten und für die Messung isometrischer Kraftparameter des Zellgewebes entfernbar sind. Ferner sind aus der DE 195 00 498 A1 ein Verfahren zum Kultivieren von Kardiomyozyten in Kollagengel, insbesondere zum meßbaren Verfolgen der Kontraktion von Herzmuskelgewebe, sowie eine Vorrichtung zum Herstellen eines Matrixkörpers aus Kollagengel und eingelagerten Zellkulturen bekannt.

Mit den in der DE 195 00 498 A1 beschriebenen Vorrichtungen und Verfahren war es erstmals gelungen, Herzmuskelzellen des Hühnchens *in vitro* zu einem künstlichen, dreidimensionalen Herzmuskelgewebe (sog. "Engineered Heart Tissue", "EHT") zu rekonstruieren. Damit lag eine dreidimensionale, hochvernetzte, elektrisch verbundene und damit den physiologischen Verhältnissen *in vivo* nahekommende Struktur vor. Davor wurden Herzmuskelzellen in einer einzelnen Schicht in Plastikschalen kultiviert (sog. zweidimensionale Kultur).

Darüber hinaus war es mit den in der DE 195 00 498 A1 offenbarten Vorrichtungen und Verfahren möglich, eine Überwucherung der Kardiomyozyten durch Fibroblasten in Anwesenheit von Serum und Wachstumsfaktoren zu unterbinden. Dies erlaubt weitgehend physiologische Wachstums- und Differenzierungsbedingungen ohne die Notwendigkeit, Zytostatika einzusetzen. In der zweidimensionalen Standardkultur kommt es normalerweise zu einer ausgeprägten Überwucherung der nicht teilungsfähigen Herzmuskelzellen durch teilungsaktive Fibroblasten, glatte Muskelzellen und Endothelzellen. In herkömmlichen zweidimensionalen Kulturen wird dies üblicherweise durch Entzug von Serum und Zugabe von Zytostatika unterbunden.

Es war ferner mit der aus der DE 195 00 498 A1 bekannten Vorrichtung möglich, Kraft, Frequenz, Kontraktionskinetik und diastolische Spannung bei Herzmuskelzellen sicher und reproduzierbar unter isometrischen Bedingungen zu messen. Dies erlaubte eine erheblich differenziertere und validere Beurteilung der Funktion von Herzmuskelzellen als dies bei neonatalen oder frisch isolierten adulten Kardiomyozyten möglich gewesen wäre. Beispielsweise ist bei neonatalen Herzmuskelzellen in einer zweidimensionalen Kultur die Kraft gar nicht und die Frequenz nur sehr eingeschränkt meßbar. Bei adulten, frisch isolierten Herzmuskelzellen kann man in einem Zeitraum von bis zu 6, maximal 24, Stunden relativ verläßlich das Ausmaß der Verkürzung als Parameter der isotonischen Kraft messen. Da sich jedoch Herzmuskelzellen normalerweise im Gewebeverband befinden, ist die Kontraktion niemals isotonisch, sondern in der Regel überwiegend isometrisch mit einer kleinen isotonischen Komponente. Gegenüber intakten Präparaten und isolierten Zellen besteht somit bei den dreidimensionalen Zellverbänden der Vorteil einer über Tage bis Wochen stabilen Situation, was besonders für genetische Manipulationen wichtig ist.

Auch erlauben die in der DE 195 00 498 A1 beschriebenen Apparate und Methoden anders als intakte Präparate eine gute mikroskopische Zugänglichkeit *in situ* und nach histologischer Färbung.

Schließlich konnte auch noch gezeigt werden, daß das EHT gemäß der zitierten deutschen Offenlegungsschrift eine exzellente Gentransfereffizienz mit Adenoviren aufweist (100% der lebenden Zellen).

Aus der EP-A-0 751 215 ist ein Gefäß zum Kultivieren von Zellen bekannt, das ein offenes Behältnis mit einer Bodenfläche umfaßt. Die Bodenfläche weist eine Vielzahl von nach oben ragenden Vorsprüngen auf. Die nach oben ragenden Vorsprünge dienen dazu, Deckgläser oberhalb der Bodenfläche zu stützen, was eine wesentlich einfachere Handhabung der Deckgläser erlaubt, wenn diese entfernt werden, da dadurch eine Haftung der Deckgläser wegen Oberflächenspannungseffekten an dem Medium und den Zellen im wesentlichen unterbunden wird.

Die Japanische Offenlegungsschrift Nr. 02/154679 beschreibt ein Untersuchungsgefäß für medizinische Zwecke. Das Gefäß enthält eine Gruppe von kleinen Erhebungen, die auf dem Boden des schalenförmigen Gefäßes an beliebigen Stellen angeordnet sind. Die Zwischenräume zwischen den Erhebungen sind so ausgestaltet, daß sich Flüssigkeit dazwischen halten kann. In entsprechenden Gefäßen kann auf einfache und genaue Weise eine Vielzahl von Reaktionen und Messungen durchgerührt werden.

Die Japanische Offenlegungsschrift Nr. 06/032345 offenbart eine Petrischale mit einer Erhebung oder mehreren Erhebungen für ein Ätzverfahren.

Die US-A-3,902,972 befaßt sich mit einer Schale, die für den Einsatz mit mechanischen Beimpfungsvorrichtungen vorgesehen ist. Die Schale ist mittels Wänden in verschiedene Bereiche unterteilt. In den verschiedenen Bereichen befinden sich verschiedene Medien.

Aus der US-A-3,540,985 ist ebenfalls eine Petrischale bekannt, in welcher mikrobilogische Testzylinder angeordnet sein können. Die entsprechende Vorrichtung ist zur Bestimmung der Wirksamkeit von Antibiotika geeignet. In der Petrischale sind Mittel vorgesehen, um die Testzylinder in fest zu positionieren.

Die US-A-3,985,608 und die US-A-4,140,582 beschreiben ein Behältnis, das für mikrobiologische Tests und andere Laborarbeiten geeignet ist. Das Behältnis weist Elemente auf, welche einzelne Bereiche in dem Behältnis abteilen. Die Elemente können mittels Magnetkraft auf dem Boden des Behältnisses befestigt werden.

Die US-A-4,975,377 beschreibt Wachstumskammern für verankerungsunabhängiges Zellwachstum in diesen Kammern. Die Wachstumskammern sind aus einer Gelmatrix aufgebaut und erlauben kein verankerungsabhängiges Zellwachstum auf deren gesamter Innenseite. Vorzugsweise sind die Kammern mit einer zylindrischen Wand ausgestattet.

Aus der EP-A-0 218 065 ist eine biologisch aktive Basalmembranzusammensetzung bekannt. Wenn diese unter physiologischen Bedingungen polymerisiert wird, bildet die Zusammensetzung gelartige Strukturen, deren Ultrastruktur verbundenen dünnen Schichten der Lamina-densa-Zone der Basalmembran ähnlich sieht. Die Hauptbestandteile der Zusammensetzung sind Laminin, Kollagen vom Typ IV, Heparansulfatproteoglycan, Entacin und Nidogen. Die rekonstituierte Matrix ist biologisch aktiv und stimuliert das Wachstum und die Differenzierung einer Vielzahl von Zellen, wie z.B. Epithelzellen, Nervenzellen, Haarfollikeln und ähnlichen. Die rekonstituierte Matrix kann auch dafür verwendet werden, um das Metastasenpotential von Tumorzellen zu bestimmen.

Trotz der vorstehend beschriebenen Vorteile und Fortschritte gegenüber einer zweidimensionalen Kultur weisen die in der DE 195 00 498 A1 offenbarten dreidimensionalen Zellverbände noch eine Reihe von Nachteilen auf. So konnte man bisher nur Hühnchenherzmuskelzellen und keine Säugerherzmuskelzellen zu dreidimensionalen künstlichen Herzgeweben gestalten. Des weiteren ist die Herstellung von EHT mittels der in der DE 195 00 498 A1 beschriebenen Vorrichtung relativ umständlich, da zuerst Röhrchen mit einem Metallbügel zusammengebaut, die Röhrchen dann mit einem Klettband beklebt sowie in Vertiefungen in einer Zellkulturschale ausgerichtet werden müssen und dann eine Kollagen/Zell-Mischung in die Vertiefungen pipettiert werden muß. Nach maximal fünfmaligem Gebrauch müssen die Vorrichtungen weggeworfen werden. Da die einzelnen Komponenten der Vorrichtungen gemäß der DE 195 00 498 A1 in Handarbeit gefertigt werden, unterliegen sie einer gewissen Variabilität, wodurch das Meßergebnis beeinflußt wird. Der Übergang zwischen Klettband und Herzgewebe entspricht des weiteren nicht den physiologischen Bedingungen. Schließlich ist die Apparatur nach der vorher genannten deutschen Offenlegungsschrift relativ umständlich zu handhaben und eine gewisse Mindestgröße ist konstruktionsbedingt nicht unterschreitbar.

Der vorliegenden Erfindung liegt somit die Aufgabe zugrunde, ein Säugerherzmuskelgewebe und ein Verfahren zü dessen Herstellung zur Verfügung zu stellen, welche die vorstehend beschriebenen Nachteile nicht aufweisen.

Es wurde nun überraschender weise gefunden, daß Muskelzellen, die unter geeigneten Bedingungen in einer Zellkulturschale mit mindestens einem in der Zellkulturschale angeordneten Element kultiviert werden, ein dreidimensionales Muskelzellgewebe um dieses Element herum ausbilden. Dieses Muskelzellgewebe ist so stabil, daß es bei Bedarf manuell herausgenommen und physiologischen, pharmakologischen und/oder biotechnologischen Versuchen unterworfen oder sogar transplantiert werden kann.

Somit kann die der vorliegenden Erfindung zugrundeliegende Aufgabe durch das Säugerherzmuskelgewebe gemäß des unabhängigen Patentanspruchs 1 und das Verfahren gemäß des unabhängigen Patentanspruchs 5 gelöst werden. Weitere vorteilhafte Ausgestaltungen, Aspekte und Details der Erfindung sind in den abhängigen Patentansprüchen, der Beschreibungseinleitung, den Beispielen sowie der Zeichnung angegeben.

Die erfindungsgemäße Vorrichtung zum Herstellen eines dreidimensionalen, zirkulären Muskelkörpers aus einer Trägersubstanz und darin eingelagerten Zellkulturen weist eine Zellkulturschale und mindestens ein Element, welches in der Zellkulturschale angeordnet ist, auf. Dadurch wird erreicht, daß die Zellverbände (z.B. EHTs) nicht mehr, wie in der DE 198 00 498 A1 beschrieben, als an zwei Röhrchen über Klettband befestigte bikonkave Körper produziert werden, sondern als kontinuierliche Ringe. Die Ringform des Gewebes ergibt sich einfach dadurch, daß eine Mischung aus Trägersubstanz und Zellen in die Zellkulturschale gegossen wird, in welcher mindestens ein Element angeordnet ist. Das EHT wächst dann als Endlosband um das Element herum. Besonders günstig ist es, wenn das Element zentral und/oder herausnehmbar in der Zellkulturschale angeordnet ist. Dabei kann das Element z.B. entweder über ein Gewinde in den Boden der Zellkulturschale einschraubbar, oder gemäß einer besonders bevorzugten Ausführungsform einfach in den Boden der Schale einsteckbar sein.

Damit sich ein dreidimensionaler, zirkulärer Muskelkörper bzw. Muskelring in der Zellkulturschale ausbilden kann, sollte das Element sich in jedem Fall über die Füllhöhe des Gemischs aus Trägersubstanz und Zellen (und u.U. einer Nährlösung) hinaus erheben. Ist die Länge oder Höhe des Elements darüber hinaus auch noch höher als der Rand der Zellkulturschale, so ist unabhängig von der Füllhöhe der Schale in jedem Falle sichergestellt, daß sich in der erfindungsgemäßen Vorrichtung ein dreidimensionaler, zirkulärer Muskelkörper ausbilden kann. Das in die Zellkulturschale einsetzbare oder integral mit dieser verbundene Element weist somit vorzugsweise eine Länge bzw. Höhe E_{H} auf, die größer ist als die Füllhöhe F_{H} des sich in der Zellkulturschale befindlichen Gemischs aus Trägersubstanz, Zellen und gegebenenfalls Nährflüssigkeit. Insbesondere ist es bevorzugt, daß die Höhe E_{H} des Elements auch größer ist als die Höhe R_{H} des Randes der Zellkulturschale.

Beim Gießen des Gemischs aus Trägersubstanz und Zellen in die Zellkulturschale breitet sich das Gemisch in dieser aus. An der Stelle, an das mindestens eine Element angeordnet ist, kann sich das Gemisch nicht verbreiten. Das künstliche Zellgewebe bildet sich um das Element herum aus. Das fertige künstliche Zellgewebe kann dann aus der Zellkulturschale entnommen werden. Ist das Element herausnehmbar, kann das künstliche Zellgewebe zusammen mit dem Element aus der Schale entnommen werden. Dies bringt eine Reihe von Vorteilen im Vergleich zur bisherigen Handhabung (wie in der DE 195 00 498 A1 beschrieben) mit sich.
(1) Der gesamte Arbeitsaufwand wird geringer, da die folgenden Arbeitsschritte wegfallen: Der Zusammenbau der Röhrchen mit einem Metallbügel, das Bekleben der Röhrchen mit Klettband, die Ausrichtung der Vorrichtung in den vorgefertigten Vertiefungen der Zellkulturschale und das mühsame Pipettieren des Gemischs aus Trägersubstanz und Zellen in diese Vertiefungen. Dadurch wird auch die Produktion größerer Testserien unaufwendiger und somit auch kostengünstiger.
(2) Die Vorrichtung kann zeitlich unbegrenzt verwendet werden, während sich die mit Klettband beklebten Röhrchen abnutzen, maximal fünfmal einsetzbar sind und danach weggeworfen und durch neue ersetzt werden müssen.
(3) Die Uniformität der ringförmigen Konstrukte ist leicht zu perfektionieren, da die erfindungsgemäße Vorrichtung maschinell und mit geringsten Toleranzen gefertigt werden kann, während die Komponenten der in der DE 195 00 498 A1 beschrieben Vorrichtung in Handarbeit gefertigt werden müssen und damit einer nicht unerheblichen Variabilität unterliegen. Darunter leidet die Reproduzierbarkeit von Versuchsserien bei Verwendung der aus der genannten Offenlegungsschrift bekannten Vorrichtung.
(4) Die ringförmigen Konstrukte, die unter Verwendung der Vorrichtung erhältlich sind, sind stabiler und auch biologisch einem natürlichen Herzen ähnlicher als frühere Zellgewebe, da der unphysiologische Übergang von Zellgewebe zu Klettband bzw. Röhrchen wegfällt.
(5) Die Handhabung der ringförmigen Konstrukte für Untersuchungen ist einfacher und leichter als bei den früher erhältlichen Zellgeweben (mit Klettband/Röhrchen).
(6) Die ringförmigen Konstrukte lassen sich leichter als die früher erhältlichen Zellgewebe (mit Klettband/Röhrchen) minaturisieren.

Das in der Zellkulturschale angeordnete Element kann auch einstückig (integral) mit der Zellkulturschale ausgebildet sein. Für die leichtere Entnehmbarkeit der Konstrukte aus der Schale ist es jedoch bevorzugt, daß das Element und die Kulturschale trennbar voneinander sind.

Vorteilhafterweise ist das in der Zellkulturschale angeordnete mindestens eine Element zylinderförmig ausgebildet, wodurch sich bei einer geeigneten Form der Zellkulturschale ein ringförmiges Konstrukt (dreidimensionaler, zirkulärer Muskelkörper) mit einer runden Öffnung in der Mitte ergibt. Der Durchmesser des zylinderförmigen Elements liegt vorzugsweise zwischen 3 und 8 mm, insbesondere bei etwa 5 mm. Weitere besonders geeignete Formen für das Element sind kegelstumpfartig oder oval. Als besonders geeignetes Material, aus dem das Element aufgebaut ist, hat sich im Falle einer einstückig ausgebildeten Zellkulturschale ein dauerelastisches Materials, wie Latex oder Silicon, erwiesen. Ist das Element entnehmbar aus der Schale, so ist dieses vorteilhafterweise aus einem nichtklebenden, autoklavierbaren, festen, nicht-elastischen Material, wie Teflon oder Delrin, aufgebaut. Teflon ist Polytetrafluorethylen (PTFE) und ist eine eingetragene Marke für DuPont. Delrin ist ebenfalls eine eingetragene Marke und über DuPont beziehbar. Delrin ist ein Polyacetalkunststoff (Polyoxymethylen), der durch Polymerisation von wasserfreiem Formaldehyd gewonnen wird und eine sehr gute mechanische, thermische und chemische Beständigkeit aufweist.

Besonders geeignet sind runde oder ellipsenförmige (ovale) Zellkulturschalen, da damit am leichtesten runde (zirkuläre) Zellgewebsverbände hergestellt werden können. Im Falle einer runden Zellkulturschale liegt deren Durchmesser vorteilhafterweise zwischen 10 und 20 mm, insbesondere bei 15 mm. Im Falle einer ellipsenförmigen (ovalen) Zellkulturschale liegt vorteihafterweise die große Hauptachse zwischen 10 und 30 mm, insbesondere bei 25 mm, und die kleine Hauptachse zwischen 5 und 20 mm, insbesondere bei 15 mm, wobei die große Hauptachse immer größer als die kleine Hauptachse ist.

Die Vorrichtung ist hervorragend zur Herstellung von künstlichem, dreidimensionalem Muskelgewebe, insbesondere Herzgewebe (EHT), geeignet. Dazu werden bevorzugt als Trägersubstanz Kollagen und als Zellen Muskelzellen, speziell Herzmuskelzellen, eingesetzt. Insbesondere bei Verwendung der nachstehend beschriebenen speziellen Lösungen können in der erfindungsgemäßen Vorrichtung auch Säugermuskelzellverbände, insbesondere Säugerherzmuskelzellverbände, gezüchtet werden. Geeignete Säuger sind beispielsweise Ratten und Mäuse. Dabei können als Ausgangsmaterial entweder direkt Muskelzellen, insbesondere Herzmuskelzellen, oder Stammzellen, die *in vitro* zu Muskelzellen, insbesondere Herzmuskelzellen, differenziert werden können, eingesetzt werden. Beispiele für die letztgenannte Alternative sind die aus pluripotenten, embryonalen Stammzellen der Maus mittels eines Verfahrens im hängenden Tropfen ("hanging-drop"-Verfahren wie im Ausführungsbeispiel des deutschen Patents DD 299 439 A5 beschrieben) und die aus adulten Stammzellen der Maus gewonnenen Zellen, die jeweils zu Muskelzellen bzw. Herzmuskelzellen differenziert werden können.

Ein weiterer wichtiger Vorteil der Vorrichtung ist die Möglichkeit, eine Vielzahl von Vorrichtungen unmittelbar neben- und/oder hintereinander anordnen zu können. Dabei ist prinzipiell jede Anzahl von nebeneinander angeordneten Vorrichtungen denkbar. Als günstig hat es sich jedoch herausgestellt, wenn 24 Vorrichtungen (4 x 6) oder ein Mehrfaches davon in einer sog. "Multi-Well-Platte" zusammengefaßt sind. Mit diesen Multi-Well-Platten ist es möglich, viele künstliche Zellgewebe (insbesondere EHTs) simultan herzustellen, d.h. diese gleichzeitig zu gießen und reifen zu lassen. Die ausgereiften Zellverbände können dann ohne zusätzlichen Manipulationen weiteren Untersuchungen unterzogen werden, im Fall von EHTs können beispielsweise online-Kraftmessungen durchgeführt werden. Geeignete Vorrichtungen hierfür werden weiter unter noch näher beschrieben.

Die Entwicklung einer Multi-Well-Platte zur Herstellung und Messung von EHTs stellt einen entscheidenden Schritt für die Verwendung der EHTs in Screening-Verfahren mit hohem Durchsatz ("high throughput screening") von Arzneimitteln im weitesten Sinne dar. Neben dem Einfluß klassischer chemischer Verbindungen können auch die Auswirkungen eines Gentransfers, beispielsweise mit rekombinanten Adenoviren, und von antisense Oligonukleotiden auf die Herzmuskelzellfunktion untersucht werden.

Ferner wird eine Lösung zum Kultivieren von Säugerkardiomyozyten in einer Trägersubstanz sowie ein Verfahren zum Kultivieren einer Zellkultur in einer Trägersubstanz unter Verwendung dieser Lösung beschrieben. Wie eingangs bereits erwähnt, war es bisher nicht gelungen, ein Medium bereitzustellen, in dem Säugerkardiomyocyten ein dreidimensionales Gewebe ausbilden. Frühere Versuche wurden daher mit Hühnchenherzmuskelzellen durchgeführt. Die Lösung zum Kultivieren von Säugerkardiomyocyten enthält einen Anteil an extrazellulärer Matrix des Engelbreth-Holm-Swarm-Tumors (kurz "Matrigel" genannt). Erhältlich ist dieses Matrigel z.B. von Harbor Bio-Products, Tebu, Frankfurt (BRD). Vorzugsweise enthält die Lösung mindestens soviel Matrigel, daß dieses im Rekonstitutionsgemisch (d.h. dem Gesamtgemisch aus Trägersubstanzlösung (wie z.B. Kollagenlösung), Zellsuspension (wie z.B. Rattenherzmuskelzellsuspension) und Nährstofflösung) in einer Konzentration von mindestens 5 Vol.-%, insbesondere zwischen 5 und 15 Vol.%, enthalten ist. Sehr gute Ergebnisse werden erzielt, wenn Matrigel im Rekonstitutionsgemisch in einer Konzentration von etwa 10 Vol.-% vorhanden ist. Dies kann beispielsweise auf folgende Weise erreicht werden. Ein zweifach konzentriertes Nährmedium (2x Dulbecco's modifiziertes essentielles Minimalmedium (2x DMEM), 20% Pferdeserum, 4% Hühnchenembryoextrakt, 200 µg/ml Streptomycin und 200 U/ml Penicillin G) wird mit einer Trägersubstanzlösung (z.B. Kollagenlösung) im Verhältnis von 50:50 Volumenteilen gemischt, so daß die Endkonzentration an Nährmedium in dem Gemisch aus Trägersubstanzlösung und Nährmedium 1x DMEM, 10% Pferdeserum, 2% Hühnchenembryoextrakt, 100 µg/ml Streptomycin und 100 U/ml Penicillin G ist. Das Gemisch wird anschließend mit wenig Lauge (z.B. 0,1 N NaOH) neutralisiert. Zu etwa 85 Vol.-% des neutralisierten Gemischs werden etwa 15 Vol.-% Matrigellösung hinzugegeben. Schließlich wird das Gemisch, das Trägersubstanzlösung, Nährmedium und Matrigel enthält, im Verhältnis von etwa 70:30 Vol.-% mit einer Zellsuspension gemischt. Das Endgemisch (Rekonstitutionsgemisch) enthält dann etwa 10 Vol.-% an Matrigel.

Es versteht sich von selbst, daß entsprechende Gemische auch andere Matrigelkonzentrationen enthalten können, solange sichergestellt ist, daß die Konzentration im Rekonstitutionsgemisch ausreichend hoch ist, um das Wachstum dreidimensionaler, künstlicher Gewebe, welche Säugerherzmuskelzellen enthalten, zu bewirken.

Mit der vorstehend beschriebenen, Matrigel enthaltenden Lösung war es erstmals möglich, mit Herzmuskelzellen von neugeborenen Ratten in einer Trägersubstanz (Kollagenmatrix) ein dreidimensionales, künstliches Herzgewebe auszubilden.

Der Vorteil von EHTs der Ratte gegenüber EHTs von Hühnchen ist die Tatsache, daß sich die erstgenannten von Säugetierzellen ableiten. Dies erhöht die Vergleichbarkeit von gewonnenen Ergebnissen mit dem Menschen. Ein weiterer Vorteil ist, daß die EHTs der Ratte deutlich, und zwar 2 bis 3 mal, stärker schlagen als die des Hühnchens. Schließlich ist das Verhältnis von aktiv entwickelter Kraft zur passiven Grundspannung bei der Ratte größer als beim Hühnchen (1:2 bis 1:1 bei der Ratte, <1:10 beim Hühnchen).

Des weiteren wird eine Vorrichtung für die Messung isometrischer Kraftparameter von Zellkulturen beschrieben. Diese Vorrichtung umfaßt eine Zellkulturschale, mindestens ein Element, welches in der Zellkulturschale angeordnet ist, eine Druckaufnahmeeinrichtung und eine Einrichtung zur Aufzeichnung von Druckänderungen. Dabei können die Zellkulturen direkt in der Zellkulturschale gezüchtet werden, und für die Kraftmessungen ist kein Transfer der Kulturen von einem Anzuchtgefäß in ein Meßgefäß mehr notwendig. Der Hauptarbeitsschritt beim Verfahren gemäß DE 195 00 498 A1, nämlich das Entnehmen der relativ fragilen Zellkultur (z.B. EHTs) aus einer Schale und Aufhängen in einem Organbad, wird überflüssig. Ferner wird eine Versuchsvariable ausgeschaltet, nämlich das Maß der Vordehnung im Organbad (Vordehnung im Sinne einer prozentualen Abweichung von der ursprünglich gegossenen Form). Auch können mit der Vorrichtung Dauermessungen der Kraft über Tage durchgeführt werden, während mit der aus der vorstehend erwähnten deutschen Offenlegungsschrift bekannten Vorrichtungen bestenfalls Messungen für einige Stunden möglich waren. Des weiteren erlaubt die Vorrichtung Kraftmessungen im Nährmedium, d.h. unter den normalen Wachstums- und Reifebedingungen, während bei der herkömmlichen Apparatur bei Messungen im Organbad eine wäßrige Lösung ohne Aminosäuren, Eiweiße, Wachstumsfaktoren etc. verwendet wird. Schließlich ist mit der neuen, Vorrichtung der instrumentelle Aufwand erheblich geringer als mit der bekannten Vorrichtung, wodurch sich auch eine wesentliche finanzielle Einsparung erzielen läßt.

Es kann des weiteren von Vorteil sein, wenn die Vorrichtung für die Messung isometrischer Kraftparameter neben den vorstehend genannten Bauteilen eine Verstärkereinrichtung aufweist, welche das Meßsignal verstärkt. Geeignete Druckaufnahmeeinrichtungen sind beispielsweise Dehnungsmeßstreifen oder Tipkatheter (z.B. Millar-Tipkatheter).

Es ist bevorzugt, wenn gemäß einer ersten Ausführungsform der Vorrichtung für die Messung isometrischer Kraftparameter die Zellkulturschale und das darin angeordnete Element einstückig ausgebildet sind, d.h. wenn das Element ein integraler Bestandteil der Zellkulturschale ist. Nachdem einer der Hauptvorteile der Vorrichtung ist, daß diese nicht mehr einen Transfer der Zellkultur in ein anderes Gefäß für Messungen erfordert, ist es auch nicht notwendig, daß das Element zusammen mit der Zellkultur aus der Kulturschale entnehmbar ist.

Wie bereits vorstehend für die Vorrichtung zum Herstellen eines dreidimensionalen Matrix- bzw. Muskelkörpers beschrieben, ist das in der Zellkulturschale angeordnete Element vorzugsweise mittig (zentral) angebracht. Soweit die Form des Elements betroffen ist, sollte diese vorzugsweise zylinderförmig oder kegelstumpfförmig sein oder einen ovalen (ellipsenförmigen) Querschnitt aufweisen. Im Falle einer zylinderförmigen Ausgestaltung weist das Element vorzugsweise einen Durchmesser im Bereich von 3 bis 8 mm, insbesondere etwa 5 mm, auf. Als Material für das Element eignet sich besonders ein dauerelastisches Material, wie beispielsweise Latex oder Silicon.

Gemäß einer zweiten Ausführungsform der Vorrichtung für die Messung isometrischer Kraftparameter von Zellkulturen erlaubt diese das Vordehnen der EHTs, bevor Messungen zur Kontraktionskraft durchgeführt werden. In diesem Fall sollte das Element aus der Zellkulturschale entnehmbar sein und aus einem festen, nicht-elastischen Materials, wie beispielsweise Teflon oder Delrin aufgebaut sein. Günstig ist es, wenn das Material auch noch nicht-klebend und autoklavierbar ist, was für Teflon und Delrin zutrifft.

Die Zellkulturschale der Vorrichtung zur Messung isometrischer Kraftparameter ist vorzugsweise rund mit einem Durchmesser von 10 bis 20 mm, insbesondere etwa 15 mm. Auch ovale (ellipsenförmige) Zellkulturschalen sind besonders geeignet, wobei in diesem Fall die große Hauptachse der Ellipse vorzugsweise zwischen 10 und 30 mm, insbesondere bei 25 mm liegt, und die kleine Hauptachse zwischen 5 und 20 mm, insbesondere bei 15 mm, liegt, wobei die große Hauptachse immer größer als die kleine Hauptachse ist.

Um die zu züchtende Zellkultur besser zu strukturieren, enthält diese vorzugsweise eine Trägersubstanz. Sind die Zellen der Zellkultur Muskelzellen, so eignet sich als Trägersubstanz insbesondere Kollagen. Speziell an Säugerherzmuskelzellen können in der Vorrichtung unter lebensnahen Bedingungen Langzeituntersuchungen zur Kraftentwicklung vorgenommen werden.

Außerdem wird auch ein Verfahren zum meßbaren Verfolgen von Kontraktionen eines in eine Trägersubstanz eingelagerten Zellgewebes beschrieben. Dabei wird zunächst eine Zellkulturschale mit einem darin angeordneten Element bereitgestellt, in welche dann ein Gemisch aus einem Trägersubstanzmaterial und einer Zellgewebesuspension eingefüllt wird. Anschließend wird die mit Träger und Zellen beschickte Zellkulturschale in einem Brutschrank inkubiert, bis der Inhalt der Zellkulturschale erstarrt ist. Vor oder auch nach diesem Inkubierungsschritt wird der Zellkulturschale eine Nährlösung zugegeben. Als nächstes wird die Zellkulturschale mit dem erstarrten Gemisch aus Trägersubstanz und Zellgewebe für mehrere Tage weiter inkubiert. Danach kann die isometrische Kraftentwicklung des Zellgewebes mittels einer Druckaufnahmeeinrichtung bestimmt und über eine Datenaufzeichnungseinrichtung aufgezeichnet werden.

Wenn die zu untersuchenden Zellen Säugerherzmuskelzellen sind, so sollte die Nährlösung eine ausreichende Menge an extrazellulärer Matrix des Engelbreth-Holm-Swarm-Tumors (Matrigel) enthalten. Geeignete Nährlösungen sind bereits oben beschrieben worden. Der Gesamtansatz enthält vorzugsweise mindestens 5 Vol.-%, insbesondere 5 bis 15 Vol.-% und speziell etwa 10 Vol.-% Matrigel. Ferner hat es sich als günstig erwiesen, wenn in dem Gemisch noch 1x Dulbecco's modifiziertes essentielles Minimalmedium (1x DMEM), 10% Pferdeserum, 2% Hühnchenembryoextrakt, 100 µg/ml Streptomycin und 100 U/ml Penicillin G enthalten sind.

Das genannte Verfahren eignet sich z.B. für Kraftmessungen an Muskelzellgewebe, wie Herzmuskelzellen. In Kombination mit Kollagen als Trägersubstanz und der vorstehend beschriebenen Lösung können mit dem erfindungsgemäßen Verfahren erstmals isometrische Kraftmessungen an künstlich hergestellten, dreidimensionalen Säugerherzmuskelzellgeweben durchgeführt werden.

Der erste Inkubierungsschritt, um das Gemisch aus Trägersubstanz und Zellsuspension zum Erstarren zu bringen, dauert in der Regel 1 bis 2 Stunden. Die Temperatur beträgt beim ersten Inkubierungsschritt vorzugsweise etwa 37°C. Abhängig von der verwendeten Zellkulturart dauert der zweite Inkubierungsschritt zwischen 2 und 15 Tagen, vorzugsweise 5 bis 12 Tage, und insbesondere für Säugerherzmuskelzellen 6 bis 10 Tage.

Bevor die in der DE 195 00 498 A1 beschriebenen Techniken zur Verfügung standen, konnte die wichtigste Herzfunktion, nämlich die Kontraktionskraft, zuverlässig nur am gesamten isolierten Herzpräparat (z.B. der Ratte, des Meerschweinchens oder des Frosches) oder an Gewebestreifen aus explantierten Tierherzen gemessen werden. Die in der genannten Offenlegungsschrift offenbarten Vorrichtungen und Verfahren ermöglichten es, Tierversuche einzusparen und vor allem die Bedeutung einzelner Proteine der Herzmuskelzelle für die Regulation der Kontraktionskraft besser untersuchen zu können. Allerdings war es bisher nicht gelungen, Säugerherzmuskelzellen zu kultivieren, und die bisher mit Hühnchenherzmuskelzellen gewonnenen Ergebnisse sind nur sehr eingeschränkt auf Säuger und insbesondere auf den Menschen übertragbar.

Die Erfindung ermöglicht nun erstmals die Züchtung von Säugerherzmuskelzellen in Gewebekultur derart, daß ein spontan und kohärent schlagender Verbund entsteht. Mit diesem Modell ist es nunmehr möglich, ganz gezielt einzelne Proteine des Säugerherzens in der Kultur in ihrer Funktion zu manipulieren und an derartig manipulierten Zellen die Kontraktionskraft zu messen.

Darüber hinaus ermöglicht die Erfindung auch erstmalig das genaue Studium der interaktiven Wirkung der verschiedenen Zelltypen im Säugerherzen auf die Kontraktionskraft. Man weiß, daß 80% der Zellen im Herzen Nicht-Muskelzellen, d.h. zum Beispiel Bindegewebszellen, Gefäßzellen oder Nervenzellen, sind. Bisher ist es nicht bekannt, welchen Einfluß diese Zellen auf die Kraftentwicklung im Säugerherzen haben. In dem durch die Erfindung vorgestellten System kann man nun verschiedene Zellpopulationen in verschiedenen Anteilen mischen und untersuchen, welchen Einfluß dies auf die Kontraktionskraft hat.

Weiterhin kann man Mechanismen der Vergrößerung von Säugerherzen (Hypertrophie) durch das aus der Erfindung resultierende System untersuchen. Dabei kann daran gedacht werden, die Zellkulturgewebe unterschiedlich lange und unterschiedlich stark vorzudehnen oder mit bestimmten Pharmaka zu inkubieren. Dies verursacht eine Vergrößerung der einzelnen Herzmuskelzellen. Im vorliegenden System ist es jetzt erstmalig möglich, auch die funktionelle Auswirkung der Vergrößerung des Säugerherzens unter den Versuchsbedingungen direkt und einfach zu messen.

Die Erfindung ermöglicht somit erstmalig die Messung der isometrischen Kontraktionskraft an embryonalen Säugerherzmuskelzellen oder, allgemeiner gesagt, an kultiviertem, vom Organismus separierten Säugermuskelgewebe. Die Erfindung ermöglicht damit die gezielte Untersuchung von Auswirkungen bestimmter Manipulationen von Säugermuskelzellen oder einer Säugerherzmuskelvergrößerung auf die Kontraktionskraft. Derartige Fragestellungen stehen im Mittelpunkt eines Großteils der experimentellen Forschung, d.h. der
- kardiovaskulären Grundlagenforschung und der
- angewandten Forschung mit dem Ziel der Entwicklung neuer herzwirksamer Pharmaka zur Therapie der Herzmuskelschwäche und von Herzrhythmusstörungen.

Es soll an dieser Stelle nochmals betont werden, daß die Herz-Kreislauferkrankungen in der westlichen Welt für die meisten Todesfälle verantwortlich sind. Die Herzmuskelschwäche ist mit einer Häufigkeit von etwa 3% der bundesdeutschen Bevölkerung eine der häufigsten Erkrankungen überhaupt. Die Behandlungsmöglichkeiten haben sich verbessert. Dennoch liegt die 5-Jahres-Sterblichkeit auch heute noch bei über 50%. Dies bedeutet, daß die Entwicklung von Medikamenten, welche die Herzmuskelschwäche verbessern können, weiter ein vorrangiges gesundheitspolitisches Ziel darstellt. Insbesondere in diesem Zusammenhang ist der Nutzen der vorliegenden Erfindung zu sehen.

Ein weiterer Aspekt der Erfindung ist, daß die Erfindung erstmals die Herstellung von künstlichem Herzgewebe der Ratte als Gewebeersatz, beispielsweise nach einem Myocardinfarkt, erlaubt. Es sind vielversprechende Ansätze vorhanden, daß die erfindungsgemäß hergestellten Ring-EHTs am Herzen anwachsen und möglicherweise die Eigenkontraktion eines erkrankten Herzens unterstützen können. Dies bietet unter Umständen ungeahnte Perspektiven zur Behandlung von Herzerkrankungen.

Die vorliegende Erfindung betrifft somit auch ein künstlich hergestelltes, dreidimensionales Muskelgewebe enthaltend eine Trägersubstanz und Muskelzellen. Das künstlich hergestellte, dreidimensionale Muskelgewebe ist erhältlich durch Herstellung eines Gemischs aus einer Trägersubstanz und einer Nährstofflösung, Neutralisierung des Gemischs, Zugabe von Matrigel und einer Muskelzellsuspension zu dem neutralisierten Gemisch sowie anschließendem Inkubieren des Gemischs unter Bedingungen, unter denen ein dreidimensionales Muskelgewebe entstehen kann. Es ist prinzipiell jede Muskelzellart einsetzbar, jedoch besonders bevorzugt, Herzmuskelzellen zu verwenden. Die Herkunft der Muskelzellen ist unkritisch. Insbesondere im Hinblick auf Erkenntnisgewinne in Zusammenhang mit Herzerkrankungen des Menschen ist es besonders bevorzugt, Säugermuskelzellen, beispielsweise aus der Ratte oder aus der Maus, einzusetzen. Es können dabei unter anderem ausdifferenzierte Muskelzellen, beispielsweise Herzmuskelzellen, als Ausgangsmaterial hergenommen werden oder auch die bereits vorstehend beschriebenen, pluripotenten, embryonalen Stammzellen der Maus, welche in spontan pulsierende Herzmuskelzellen differenzieren können (siehe DD 299 439 A5). Als bevorzugtes Trägermaterial bietet sich wiederum Kollagen an, insbesondere wenn Herzmuskelzellen oder Zellen, die zu Herzmuskelzellen differenzieren können, zum Einsatz kommen. Die übrigen Parameter (Art und Konzentration der Nährlösung, der Trägersubstanz, des Neutralisationsmittels, des Matrigels und der Muskelzellsuspension, Dauer und Temperatur des Inkubationsschrittes etc.) entsprechen vorzugsweise den in den Ansprüchen, der Beschreibungseinleitung und den Beispielen der vorliegenden Anmeldung wiedergegebenen Angaben.

Nachfolgend wird die Erfindung anhand der Figuren und Beispiele näher erläutert.

Es zeigen
- Fig. 1: eine Zellkulturschale mit einem darin angeordneten Element;
- Fig. 2: eine größere Schaleneinheit, in der mehrere Zellkulturschalen nach Fig. 1 enthalten sind;
- Fig. 3: ein mittels der in Fig. 1 gezeigten Zellkulturschale erhaltenes, ringförmiges, künstliches, dreidimensionales Herzgewebe (EHT);
- Fig. 4: einen Querschnitt durch eine Ausführungsform einer Multi-Well-Platte mit einer Vorrichtung zur Kraftmessung;
- Fig. 5: einen Querschnitt durch eine weitere Ausführungsform einer Zellkulturschale mit einer Vorrichtung zur Messung der Kontraktionskraft, gezeigt zum Zeitpunkt des Gießens des EHTs;
- Fig. 6: die Zellkulturschale gemäß Fig. 5 nach Entfernen des Platzhalters;
- Fig. 7: die in Fig. 5 gezeigte Zellkulturschale mit einer motorisierten Einrichtung zum Dehnen des EHTs;
- Fig. 8: die in Fig. 5 gezeigte Zellkulturschale mit einer Einrichtung zur Messung der Kontraktionskraft des EHTs;
- Fig. 9: eine Einrichtung zur Messung der Kontraktionskraft, wie sie in Fig. 8 verwendet wird;
- Fig. 10: ein Diagramm, aus dem die Abhängigkeit der Ratten-EHT-Bildung von der Matrigel-Konzentration in dem Rekonstitutionsgemisch ersichtlich ist;
- Fig. 11: ein Diagramm, welches die Abhängigkeit der Kontraktionskraft von EHTs von der Vordehnung der EHTs zeigt;
- Fig. 12A: einen Querschnitt durch eine Ausführungsform einer kombinierten Vorrichtung zum Herstellen eines dreidimensionalen zirkulären Muskelkörpers mit einer Vorrichtung für die Messung isometrischer Kraftparameter des Muskelkörpers; und
- Fig. 12B: einen Querschnitt durch eine Vorrichtung zur Druckerzeugung.

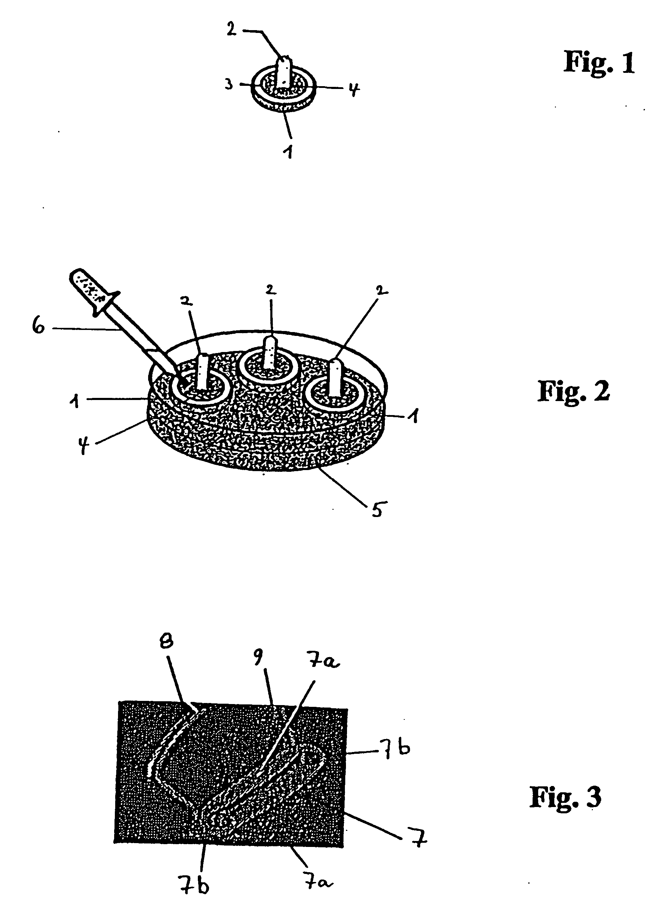

In Fig. 1 ist eine runde Zellkulturschale 1 gezeigt, welche ein zentral angeordnetes, zylinderförmiges Element 2 enthält. Die Zellkulturschale 1 weist einen erhöhten Randbereich 3 und einen Vertiefungsbereich 4 auf, der ein Gemisch aus Trägermaterial, Zellsuspension und Nährmedium aufnehmen kann. Der Durchmesser der Zellkulturschale 1 beträgt 15 mm und der Durchmesser des zentral angeordneten, zylindrischen Elements beträgt 5 mm. Wie aus Fig. 1 ersichtlich ist, ragt das zentrale Element 2 über den erhöhten Randbereich 3 der Zellkulturschale 1 hinaus. Die Höhe bzw. Länge des Elements E_{H} ist somit größer als die Randhöhe R_{H} der Zellkulturschale 1, womit in jedem Falle sichergestellt ist, daß sich in der Zellkulturschale ein dreidimensionales, ringförmiges bzw. zirkuläres Muskelzellgewebe ausbilden kann.

Drei der in Fig. 1 gezeigten Zellkulturschalen 1 sind in Fig. 2 in einer größeren Schaleneinheit 5 angeordnet. Die Schaleneinheit 5 ist eine handelsübliche Petri-Schale aus Kunststoff mit einem Durchmesser von 10 cm. In einer solchen Schaleneinheit 5 können bis zu 5 Zellkulturschalen 1 untergebracht werden. Des weiteren ist in Fig. 2 eine Pipette 6 gezeigt, mit der eine Zellsuspension/Kollagen-Mischung in eine Zellkulturschale 1 pipettiert wird.

In Fig. 3 ist ein künstliches, dreidimensionales, ringförmiges Herzgewebe (EHT) 7 gezeigt, wie es nach etwa 8 Tagen Inkubation in einer Zellkulturschale 1, welche mit einer Mischung aus Kollagen, einer Rattenherzmuskelzellsuspension, Matrigel und Nährmedium beschickt worden ist, erhalten wurde. Das EHT ist zwischen zwei Haltedrähten 8, 9 aufgespannt und hat dadurch seine ursprüngliche Kreisform verloren. Das in Fig. 3 gezeigte EHT weist längere, parallele Abschnitte 7a und enge, bogenförmige Abschnitte 7b auf, wobei sich die Abschnitte 7b um die Haltedrähte 8, 9 herum erstrecken.

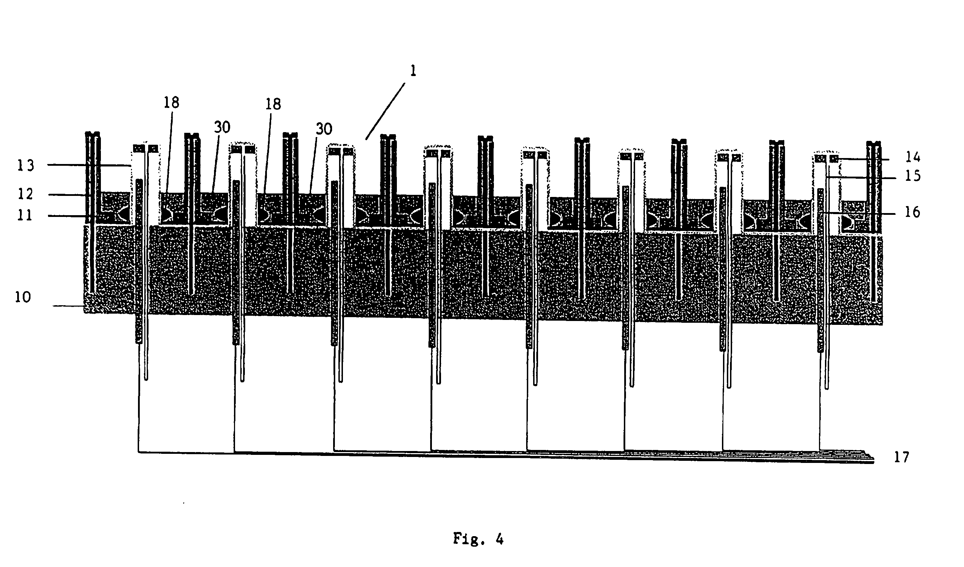

Fig. 4 zeigt einen Querschnitt durch eine Multi-Well-Platte, mittels der eine Druckmessung an den in Fig. 3 gezeigten ringförmigen EHTs (in Fig. 4 mit der Bezugsziffer 18 bezeichnet) durchgeführt werden kann. Die einzelnen EHTs befinden sich in einem Nährmedium 30. Mit der Bezugsziffer 10 ist ein sog. Delrin- Träger bezeichnet, welcher als Grundplatte der Multi-Well-Platte fungiert, und Bezugsziffer 11 stellt eine Teflonmaske dar. Mit der Schraube 12 aus V2A-Stahl ist die Teflonmaske 11 mit dem Delrin-Träger 10 verschraubt. Zentral in der Zellkulturschale 1 ist ein zylindrisches, dauerelastisches Element 13 aus Latex angeordnet. Das Element 13 weist die Form eines Hohlkörpers auf. In diesem Hohlkörper befindet sich ein Träger 14, welcher den Hohlkörper formstabil hält. Des weiteren treten von unten durch den Delrin-Träger 10 eine Entlüftungskanüle 15 und ein Millar-Tipkatheter 16 als Druckaufnahmeeinrichtung in das hohle Element 13 ein. Von den Tipkathetern 16 gehen Leitungen 17 über einen nicht gezeigten Verstärker zu einer ebenfalls nicht gezeigten Aufzeichnungseinrichtung (einem Personal Computer).

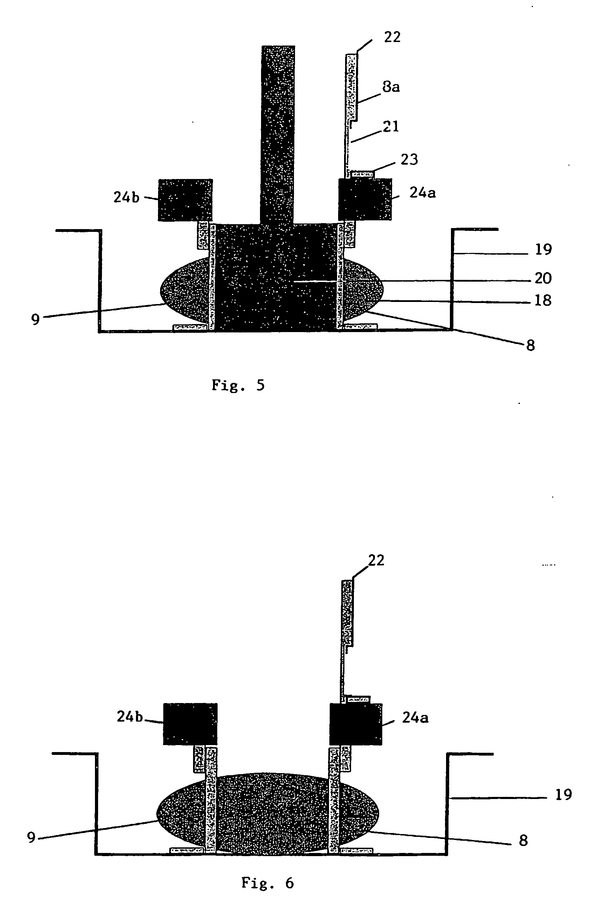

Gemäß einer alternativen Ausführungsform wird bei den erfindungsgemäß hergestellten EHTs nicht der entwickelte Druck, sondern direkt die isometrische Kraft gemessen. Dazu wird ein EHT-Ring 18 in einer Zellkulturschale 19 wie in Fig. 5 gezeigt um ein Element 20 herum gegossen, welches eine zylindrische Form aufweist, zentral in der Zellkulturschale 19 angeordnet ist und aus einem festen, nicht-elastischen Material (Teflon) besteht. Das zylindrische Element 20 kann nach der Bildung der EHTs durch Herausziehen nach oben aus der Zellkulturschale 19 entfernt werden. Fig. 6 zeigt die Zellkulturschale 19 ohne zylindrisches Element 20. Das EHT wird nun von zwei Haltedrähten 8 und 9 gehalten (siehe Fig. 3,5 und 6). Die Haltedrähte 8 und 9 sind während des Gießens und des Wachstums der EHTs in zwei Ausnehmungen des zylindrischen Elements 20 angeordnet, so daß die Außenkontur des zylindrischen Elements 20 trotz des Vorhandenseins der beiden Haltedrähte 8 und 9 einer glatten Zylinderwand entspricht.

In Fig. 5 ist ferner eine Einrichtung gezeigt, mit der die entwickelte Kontraktionskraft der EHTs gemessen werden kann. Diese Einrichtung weist einen im mittleren Bereich 8a des Haltedrahts 8 angeordneten Dehnungsmeßstreifen 21 auf, welcher über einen Kabelanschluß 22 und einen nicht gezeigten Verstärker mit einer ebenfalls nicht gezeigten Aufzeichnungseinrichtung in Verbindung steht. Der Haltedraht 8 weist stabile (starre) untere und obere Bereiche 8b, 8c auf, welche über einen flexiblen Teil (mittlerer Bereich 8a) miteinander in Verbindung stehen. Der flexible, mittlere Teil 8a des Haltedrahts 8 besteht aus einem dauerelastischen Material (im vorliegenden Fall aus dauerelastischem Federstahl), die stabilen (starren) oberen und unteren Abschnitte 8b, 8c des Haltedrahts 8 aus V2A-Stahl. Der Dehnungsmeßstreifen 21 ist auf den flexiblen, mittleren Teil 8a des Haltedrahts 8 aufgeklebt und registriert die durch Kontraktionen der Zellen verursachten Verbiegungen des flexiblen Teils des Haltedrahts.

Mit der Bezugsziffer 23 ist in Fig. 5 eine Arretierung des Haltedrahts an einer Dehnungsstange 24a bezeichnet. Mit dieser Anordnung kann nun die Kontraktionskraft von EHTs direkt am Ort ihrer Herstellung bestimmt werden, ohne diese, wie früher nötig (siehe DE 195 00 498 A1), aus der Anzuchtschale entnehmen und in ein Organbad zur Messung überführen zu müssen.

Von der in Fig. 5 gezeigten Zellkulturschale 19 kann eine Vielzahl hinter- und/oder nebeneinander angeordnet sein, so daß auch diese Ausführungsform der Erfindung in Form von Multi-Well-Platten ausgestaltet sein kann. Auch hier sind wieder 24 (4 x 6) oder ein Vielfaches davon an neben- und hintereinander angeordneten Zellkulturschalen bevorzugt.

Wie bereits vorstehend erwähnt, stellt Fig. 5 die Situation nach dem Gießen der EHT-Ringe 18 um das zylindrische Element 20 in der Zellkulturschale 19 dar. Die EHTs können daraufhin für einige (ca. 3 bis 4) Tage ausreifen. Danach wird, wie aus Fig. 6 ersichtlich, das zylindrische Element 20 entfernt, und die EHT-Ringe werden nunmehr von den Haltedrähten 8 und 9 innen gehalten. Die beiden Haltedrähte werden dann soweit voneinander entfernt, bis sich der EHT-Ring 7, wie in Fig. 3 gezeigt, als ein Längsoval erstreckt, das zwei parallel angeordnete Bereiche 7a aufweist. In dieser Position kann die Kraft, die das EHT entwickelt, direkt durch Biegung des Haltedrahts 8 ermittelt werden (in der beschriebenen Ausführungsform wird die Kraft, wie oben angegeben, über den Haltedraht 8 gemessen). Dazu ist der Haltedraht 8 in seinem mittleren, flexiblen Bereich 8a mit einem Dehnungsmeßstreifen 21 beklebt, der über eine Verstärkermeßbrücke (nicht gezeigt) mit einem Aufzeichnungsgerät (Personal Computer, ebenfalls nicht gezeigt) verbunden ist. Der flexible Bereich 8a erlaubt es dadurch eine Kraftmessung an einzelnen EHTs durchzuführen.

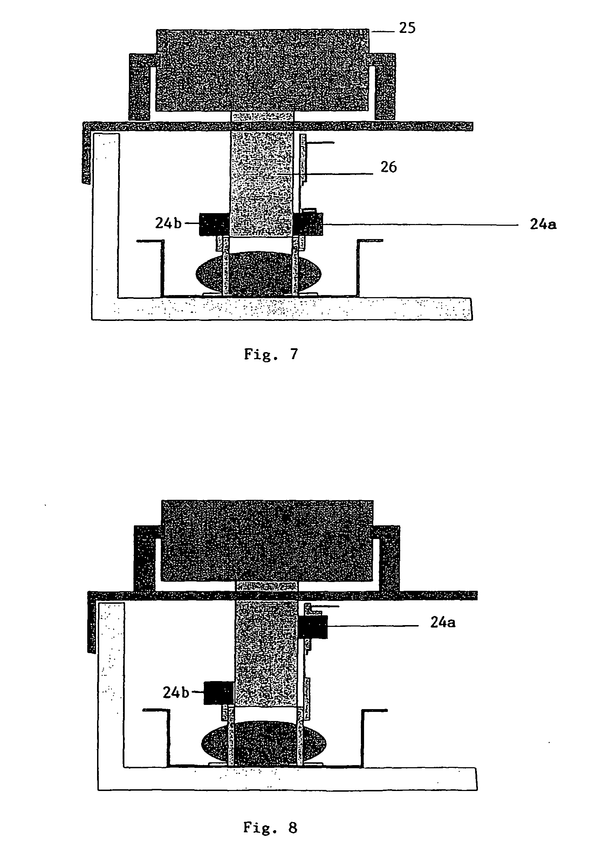

Um optimale Kraftausbeuten zu erzielen, ist es vor einer Messung der Kontraktionskraft der EHTs notwendig, diese vorzudehnen. Eine entsprechende Dehnungsmotorik ist in Fig. 7 gezeigt. Es hat sich in den letzten Jahren herausgestellt, daß die chronische Dehnung der EHTs zu einer erheblichen Zunahme der Gewebeentwicklung und Größenzunahme der einzelnen Herzmuskelzellen sowie zu einer Zunahme der kontraktilen Kraft auf das drei- bis fünffache im Vergleich zu nicht vorgedehnten EHTs führt. Es ist daher günstiger, wenn die Kraftmessung nicht unmittelbar nach Ausreifung der EHTs vorgenommen wird. Die positiven Wirkungen der Vordehnung treten bereits nach etwa 24 Stunden auf. Üblicherweise wird die Vordehnung über einen Zeitraum von etwa 1 bis etwa 7 Tage durchgeführt, da eine Vordehnung, die länger als 7 Tage dauert, praktisch keine Erhöhung der Kontraktionskraft mehr mit sich bringt. Das Ausmaß der Vordehnung sollte im Bereich von etwa 3 bis etwa 20 % der Ausgangslänge liegen, wobei eine Vordehnung von etwa 10% besonders bevorzugt ist.

Günstigerweise wird die Vordehnung motorisiert vorgenommen. Im Zustand des Gießens und der motorisierten Dehnung der EHTs befinden sich die Dehnungsstangen 24a, 24b, an denen die Haltedrähte 8 und 9 befestigt sind, in einer unteren Stellung. Im Zustand der Kraftmessung befindet sich die Dehnungsstange 24a, an welcher der Haltedraht 8 mit dem flexiblen Anteil 8a befestigt ist, in einem angehobenen Zustand (Fig. 8), in dem der obere stabile (starre) Teil 8c des Haltedrahts an der Dehnungsstange 24a befestigt und der flexible (mittlere) Anteil 8a frei beweglich ist.

In dem in Fig. 7 gezeigten Zustand (mit in unterer Stellung befindlicher Dehnungsstange 24a) ist die Dehnung des EHTs über einen gewünschten Zeitraum (bei Ratten-EHT in der Regel 1 bis 7 Tage) möglich. In dieser Phase bleibt die Dehnungsstange 24a tiefgestellt, so daß diese unterhalb des flexiblen Anteils 8a des Haltedrahts 8 angreift, wodurch auf den flexiblen Anteil des Haltedrahts keine Kraft einwirkt. In Fig. 8 ist die Dehnungsstange 24a über den flexiblen Anteil 8a des Haltedrahts 8 hinweg angehoben worden, so daß der flexible Anteil des Haltedrahts 8 freigegeben wird und über diesen die von den EHTs entwickelte Kraft (Biegung des flexiblen Anteils 8a mit Dehnungsmeßstreifen 21) gemessen werden kann. Im Prinzip kann zwischen Dehnungsphase (Dehnungsstange 24a unten) und Meßphase (Dehnungsstange 24a oben) beliebig oft hin und her gewechselt werden. Die Applikation von Testsubstanzen, Viren, Oligonukleotiden etc. kann in jeder Phase erfolgen.

Im Falle einer Multi-Well-Platte kann, sofern genügend Kapazitäten an Aufzeichnungsmöglichkeiten vorhanden sind, die Kraftmessung in allen einzelnen Kulturschalen (d.h. auf allen Kanälen) simultan erfolgen. Ist die Speicherkapazität geringer oder soll der technische Aufwand vermindert werden, ist es auch möglich, immer nur einige Kanäle (z.B. 4 bis 8) gleichzeitig zu messen, wobei zwischen den einzelnen Kanälen der gesamten Multi-Well-Platte durch eine geeignete Software umgeschaltet werden kann. Dadurch wird, mit gewissen Lücken, eine kontinuierliche Aufzeichnung aller Kanäle über einen beliebigen Zeitraum möglich.

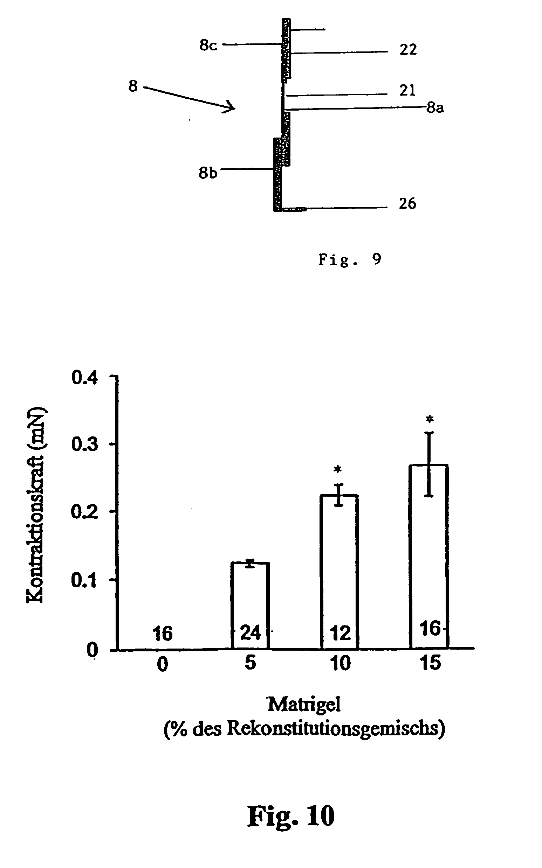

In Fig. 9 ist der Haltedraht 8 nochmals im Detail gezeigt. Der Haltedraht 8 ist im unteren Bereich steif und mit einer Sicherung 26 gegen Abrutschen versehen. In dem Bereich des Haltedrahts, in dem der Dehnungsmeßstreifen 21 angeordnet ist, ist der Haltedraht 8 flexibel. Oberhalb dieses flexiblen Abschnitts schließt sich wieder ein steifer Abschnitt an, über den der Kabelanschluß 22 zu Verstärker und Aufzeichnungseinrichtung verläuft.

### Beispiel 1

Es wurden von 0 bis 3 Tage alten, neugeborenen Ratten (insgesamt 2560 Einzeltiere) Kardiomyocyten aus den Herzen gewonnen, und zwar mittels einer Modifikation des von Webster KA, Discher DJ, Bishopric NH, 1993, Induction and nuclear accumulation of fos and jun proto-oncogens in hypoxic cardiac myocytes, J Biol Chem 268:16852-16858, beschriebenen Verfahrens. Die Zellen wurden für 1 bis 2 h in 10% fötalem Kälberserum vorplattiert, und nicht-angewachsene Zellen wurden pelletiert und in Kulturmedium suspendiert (Dulbecco's modifiziertes essentielles Minimalmedium (DMEM), 10% Pferdeserum, 2% Hühnchenembryoextrakt, 100 µg/ml Streptomycin und 100 U/ml Penicillin G). Zum Gießen der EHTs betrug die Zelldichte 10 x 10⁶ Zellen/ml. Die prinzipielle Technik wurde in Eschenhagen T, Fink C, Remmers U, Scholz H, Wattchow J, Weil J, Zimmermann H, Dohmen HH, Schäfer H, Bishopric N, Wakatsuki T, Elson EL, 1997, Three-dimensional reconstitution of embryonic cardiomyocytes in a collagen matrix: A new heart muscle system. FASEB J 11:683-694 bereits beschrieben.

Um geeignete Kulturbedingungen für Ratten-EHTs definieren zu können, wurden die folgenden Parameter untersucht:
(1) Zellisolierungsverfahren (Trypsin, verschiedene Kollagenasetypen, Dispase)
(2) Zelldichte (0,8 - 1,3 x 10⁶/EHT)
(3) Kollagenkonzentration (0,8 - 1,3 mg/ml)
(4) Ersatz des Zell/Kollagen-Rekonstitutionsgemisches mit extrazellulärer Matrix aus dem Engelbreth-Holm-Swarm Tumor (Harbor Bio-Products Tebu, Frankfurt (BRD); nachfolgend als Matrigel bezeichnet) und Serumkomplementen aus Hühnchenembryonen und/oder Säugetier.

Nach dem Standardverfahren wurde eine Zell/Kollagengemisch-Stammlösung für 8 EHTs hergestellt und bis zum Gießen auf Eis gelagert. 1,33 ml des Kollagentyps I (aus Rattenschwänzen; 3,6 mg/ml in 0,1% Essigsäure; Upstate Biotechnology, Lake Placid NY) wurden mit 1,33 ml 2x konzentriertem Kulturmedium (2x DMEM, 20% Pferdeserum, 4% Hühnchenembryoextrakt, 200 µg/ml Streptomycin, 200 U/ml Penicillin G) gemischt und mit 182 µl 0,1 M NaOH neutralisiert. Diesem Gemisch wurden 0,48 ml Matrigel zugesetzt und dem Ganzen wurden 1,48 ml der Zellsuspension zugegeben, was insgesamt 15 x 10⁶ Zellen entspricht.

1 ml der Zell/Kollagenmischung wurde in eine Zellkulturschale (Delrin) mit einem Durchmesser von 15 mm geschüttet. Das zentral in der Schale angeordnete, zylinderförmige Element hatte einen Durchmesser von 5 mm und konnte mittels einer Schraube am Boden der Zellkulturschale befestigt werden. Das Gemisch wurde dann für 60 min bei 37°C inkubiert bis es gelartig erstarrt war. Danach wurde 20 ml Kulturmedium zugegeben. Nach einer weiteren Inkubation über Nacht wurde das Medium ausgetauscht und danach jeden Tag.

Die weiteren Untersuchungen haben ergeben, daß es die Zugabe von Matrigel war, welche bewirkte, daß sich spontan und kohärent schlagende Ratten-EHTs bildeten, wobei diese Wirkung durch die Zugabe von Hühnchenembryoextrakt noch verstärkt wurde. In Fig. 10 ist dabei der Einfluß von steigenden Konzentrationen Matrigel im Rekonstitutionsgemisch gezeigt. Es wurden Ratten-EHTs hergestellt in Abwesenheit (0%) und in Gegenwart von steigenden Konzentrationen Matrigel (5%, 10%, 15% des Rekonstitutionsgemischs). Nach neuntägiger Kultivierung wurde die Kontraktionsamplitude (Kontraktionskraft) mittels einer isometrischen Kraftübertragungseinrichtung bestimmt, nachdem auf Lₘₐₓ (Länge der maximalen Kraftentwicklung) eingestellt wurde. Ratten-EHTs ohne Matrigel schlugen nicht kohärent und entwickelten somit keine meßbare Kontraktionsamplitude. Um kohärent schlagende EHTs zu bekommen, war es also notwendig, dem Rekonstitutionsgemisch Matrigel zuzusetzen. Die Kontraktionsamplitude nahm mit steigenden Matrigelkonzentrationen zu, wie in dem in Fig. 10 gezeigten Diagramm zu sehen ist. In dem Diagramm ist auf der Ordinate die Kontraktionskraft in mN und auf der Abszisse des prozentuale Anteil von Matrigel am Rekonstitutionsgemisch angegeben. Mit dem Stern über den Säulen ist eine Standardabweichung P < 0,05 gegen 5% bezeichnet. Die in den Säulen bzw. oberhalb der Abszisse angegebenen Zahlen entsprechen den untersuchten, unabhängigen EHTs.

### Beispiel 2

In diesem Beispiel soll der Einfluß der sechs Tage währenden phasischen Dehnung auf die von ETHs entwickelte Kontraktionskraft beschrieben werden.

EHTs wurden wie vorstehend beschrieben vier Tage lang kultiviert und dann für sechs Tage einer automatisierten, mittels eines Motors durchgeführten Dehnung unterzogen. Das Ausmaß der Dehnung lag zwischen 1 und 20% der Originallänge der EHTs, wobei eine weitere, nicht gedehnte Gruppe von EHTs unter ansonsten identischen Bedingungen gehalten wurde, die als Kontrollgruppe diente. Die Frequenz, mit der sich die Dehnungseinrichtung drehte, betrug 1,5 Hz. Die gesamte Vorrichtung wurde in einem CO₂-Inkubator bei 37°C gehalten. Das Kulturmedium wurde jeden Tag gewechselt.

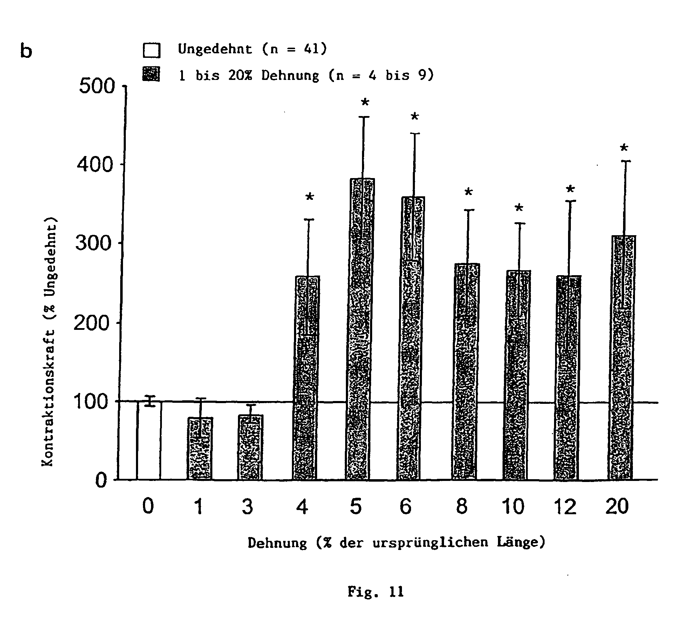

Wie aus Fig. 11 ersichtlich ist, ist erst oberhalb von 3% Dehnung ein signifikanter Anstieg der Kontraktionskraft erkennbar, der bei 5% Dehnung ein Maximum erreicht.

### Beispiel 3

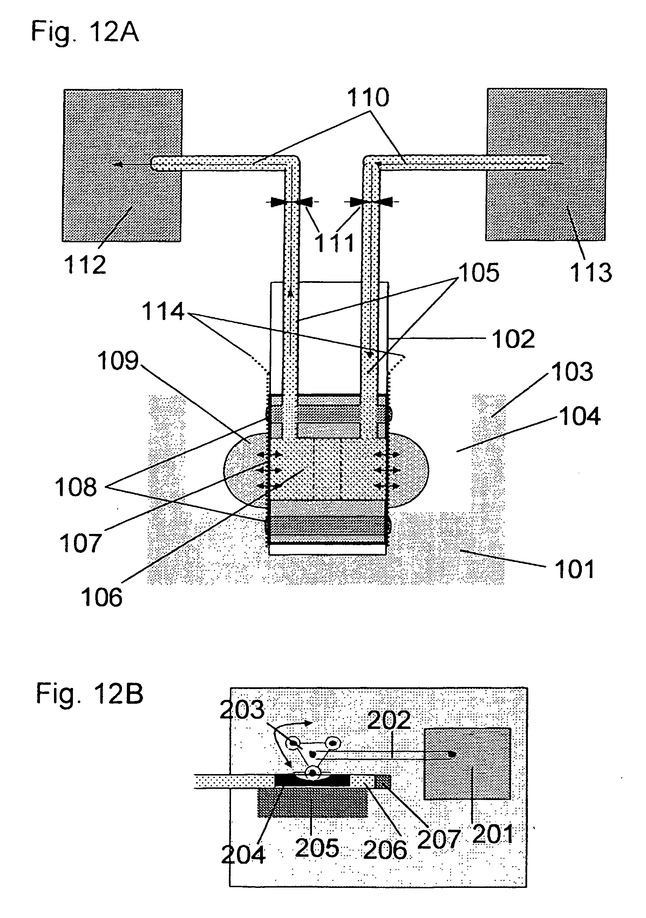

In Fig. 12A ist eine erfindungsgemäße, runde Zellkulturschale 101 gezeigt, welche ein zentral angeordnetes, zylinderförmiges Element 102 enthält. Die Zellkulturschale 101, gefertigt aus Teflon, weist einen erhöhten Randbereich 103 und einen Vertiefungsbereich 104 auf, der ein Gemisch aus Trägermaterial, Zellsuspension und Nährmedium aufnehmen kann. Andere geeignete Materialien für die Kulturschale sind z.B. Silikon oder andere Kunststoffe. Der Durchmesser der Zellkulturschale beträgt 15 mm und der Durchmesser des zentral angeordneten, zylindrischen Elements 5 mm.

Das zylinderförmige Element 102 besteht aus einem festen Kunststoff und enthält Bohrungen 105 sowie einen nach außen offenen Hohlraum 106, die mit einer Flüssigkeit gefüllt sind. Im unteren Abschnitt des zylinderförmigen Elements 102 ist eine schlauchförmige, für die Flüssigkeit undurchlässige, jedoch elastische Membran 107 um das Element 102 gestülpt, die den offenen Hohlraum 106 nach außen abdichtet. Die elastische Membran ist aus Silikon gefertigt. Ein anderes geeignetes Material für die elastische Membran ist z.B. Latex. Die Elastizität der Membran 107 ist in Fig. 12A durch die Pfeile im unteren Bereich des zylinderförmigen Elements 102 angedeutet. Die Membran ist über Befestigungen 108 fest mit dem zylinderförmigen Element 102 verbunden. Die Flüssigkeit weist eine geringe Viskosität und Dichte auf.

Die Bezugsziffer 109 bezeichnet ein künstliches, ringförmiges Herzgewebe (EHT), wie es sich nach etwa 8 Tagen Inkubation in der Zellkulturschale 101 um das zylindrische Element 102 herum gebildet hat, wobei die Zellkulturschale mit einer Mischung aus Kollagen, einer Rattenherzmuskelsuspension, Matrigel und Nährmedium beschickt worden war.

Die Bohrungen 105 und der mit der Membran 107 verschlossene Hohlraum 106 bilden zusammen mit den auf den Bohrungen aufgesetzten Röhren oder unelastischen Schläuchen 110 ein hydraulisches System. Die Pfeile in den unelastischen Schläuchen 110 geben die Fließrichtung der Flüssigkeit an. Zu dem System gehören des weiteren die Sperrventile 111, eine Vorrichtung zur Druckmessung 112 sowie eine Vorrichtung zur Druckerzeugung 113.

Die Vorrichtung zur Druckerzeugung 113 kann beispielsweise eine hydraulische Pumpe sein , wie sie in Fig. 12B dargestellt ist. Die Druckerzeugungsvorrichtung 113 weist einen Motor 201, eine Einrichtung zur Kraftübertragung 202, ein rotierendes Rollensystem 203, einen flexiblen Schlauch 204, ein Widerlager 205 und einen unelastischen Schlauch 206 auf, dessen Ende mit einem Verschluß 207 versehen ist. Anstelle des unelastischen Schlauchs kann auch eine Röhre verwendet werden. Der gebogene Pfeil in Fig. 12B gibt die Drehrichtung des Rollensystems 203 an.

Das System (Modell) funktioniert folgendermaßen. Wird das Sperrventil 111 des ableitenden (in Fig. 12A links gezeigten) Schenkels des Schlauchsystems 110 bei geöffnetem Ventil im rechten Schenkel geschlossen, so kann über die Vorrichtung zur Druckerzeugung 113 im hydraulischen System ein Druck erzeugt werden, der zu einer Ausdehnung der Membran 107 führt, welche den Hohlraum 107 flüssigkeitsdicht abschließt. Durch rhythmische Druckerzeugung kann somit der Muskelring 109 überstreckt werden.

Wird das Sperrventil 111 des zuleitenden (in Fig. 12A rechts gezeigten) Schenkels geschlossen und das sich im ableitenden (rechten) Schenkel befindliche Ventil geöffnet, so kann die Kraft des sich kontrahierenden Muskelrings (EHTs) 109 über die Volumenverkleinerung des Hohlraums 106 quantitativ in der Vorrichtung zur Druckmessung 112 erfaßt werden.

Dieses System (Modell) besitzt somit den Vorteil, daß die Herstellung und das Überstrecken des Muskelrings sowie die Kraftmessung in ein und derselben Vorrichtung erfolgen kann, ohne daß die Muskelringe von einer Gießkammer in eine Überstreck- oder Meßkammer überführt werden müßten.

Im Gegensatz zu einer Anordnung, bei der ein Muskelring zwischen zwei Haken oval aufgespannt wird und bei der somit die sich an den Enden des Muskelrings befindlichen Zellen senkrecht zur Überstreck-/Meßrichtung gedehnt werden bzw. sich kontrahieren, kann bei einer vollständig kreisförmigen Anordnung des Muskelrings jede Zelle gedehnt werden bzw. zur Volumenverkleinerung des Rings beitragen. Der entstehende Muskelring ist daher homogener und spiegelt somit eher den natürlichen Muskel wieder. Die Kraftmessung wird genauer, da im Vergleich zur herkömmlichen Apparatur zum einen die Kontraktion von mehr (und im Idealfall von allen) Zellen des Rings gemessen werden kann, und zum anderen die Kontraktion von Zellen, die sich senkrecht zur Meßrichtung kontrahieren und dadurch zu unerwünschten Schwingungen führen, vermieden werden kann.

Die erfindungsgemäße Anordnung kann miniaturisiert werden und mehrere der Anordnungen beispielsweise in 24- oder 48-Well-Zellkulturschalen zusammengefaßt werden. Durch parallele Verschaltung oder Verschaltung in Reihe solcher Anordnungen können Meßsignale gemittelt und/oder verstärkt werden.

In einer besonderen Ausführungsform kann die Membran 107 eine Piezo-Folie sein, die bei Kontraktion eine zur Kraft proportionale Spannung induziert, die wiederum mittels Elektroden gemessen werden kann. Eine derartige Kraftmessung hat gegenüber der hydraulischen Messung Vorteile in Bezug auf Sensitivität und Trägheit.

Ferner ist es möglich, das hydraulische System ganz zu ersetzen, indem man anstelle des Hohlraums 106 einen Körper anordnet, der sein Volumen in Abhängigkeit von einer angelegten Spannung verändert, die über Elektroden angelegt bzw. gemessen werden kann. Die Überstreckung des Muskelrings erfolgt durch rhythmisches Anlegen einer Spannung, die zu einer Ausdehnung des Körpers in Richtung des Muskelrings führt. Die Kraftmessung wiederum erfolgt über eine Messung der über die Volumenverkleinerung induzierten Spannung. Ein derartiges System kann weiter miniaturisiert werden und ist einfacher herzustellen und besser wiederzuverwenden als bisher eingesetzte Systeme.

Zusätzlich können Elektroden 114 angebracht werden, mit deren Hilfe die Muskelzellen elektrisch zur Kontraktion veranlaßt werden können. Bei Herzmuskelzellen kann zum Beispiel die elektrische Reizung anstelle der mechanischen Überdehnung zur Reifung des Muskelrings eingesetzt werden. Da Skelettmuskelzellen und glatte Muskelzellen im Gegensatz zu Herzmuskelzellen nicht spontan kontrahieren, kann die elektrische Reizung zur Reifung der Muskelringe sowie zur Induktion der Kontraktion eingesetzt werden. Die Kraftmessung kann dann wie vorstehend beschrieben erfolgen.

## Patentansprüche

1. Künstlich hergestelltes, dreidimensionales Säugerherzmuskelgewebe, enthaltend eine Trägersubstanz und Muskelzellen, erhältlich durch
- Herstellen eines Gemisches aus einer Trägersubstanzlösung und einer Nährstofflösung,
- Neutralisieren des Gemisches,
- Zugabe von extrazellulärer Matrix aus dem Engelbreth-Holm-Swarm-Tumor, bekannt als Matrigel^{™} und einer Muskelzellsuspension zu dem Gemisch, wobei das Gesamtgemisch aus Trägersubstanzlösung, Zellsuspension und Nährlösung mindestens 5 Vol.-% Matrigel enthält,
- Inkubieren des Gemisches unter Bedingungen, so daß ein künstliches, dreidimensionales Muskelgewebe entsteht, und
- Vordehnen des gebildeten Muskelgewebes um 3 bis 20 % der Ausgangslänge.

2. Künstlich hergestelltes, dreidimensionales Säugerherzmuskelgewebe nach Anspruch 1, **dadurch gekennzeichnet, daß** das gebildete Muskelgewebe um 10 % der Ausgangslänge vorgedehnt wird.

3. Künstlich hergestelltes, dreidimensionales Säugerherzmuskelgewebe nach Anspruch 1 oder 2, **dadurch gekennzeichnet, daß** die Vordehnung über einen Zeitraum von 1 bis 7 Tagen durchgeführt wird.

4. Künstlich hergestelltes, dreidimensionales Säugerherzmuskelgewebe nach einem der Ansprüche 1 bis 3, **dadurch gekennzeichnet, daß** das Gesamtgemisch aus Trägersubstanzlösung, Zellsuspension und Nährlösung zwischen 5 und 15 Vol.-% Matrigel enthält.

5. Verfahren zum Kultivieren einer Zellkultur in einer Trägersubstanz, wobei eine Trägersubstanz, eine Zellsuspension und eine Nährlösung enthaltend die extrazelluläre Matrix des Engelbreth-Holm-Swarm-Tumors (Matrigel) zusammengegeben werden, **dadurch gekennzeichnet, daß** die Zellsuspension Kardiomyozyten enthält und das Gesamtgemisch aus Trägersubstanzlösung, Zellsuspension und Nährlösung eine Konzentration an Matrigel aufweist, die ausreicht, um ein dreidimensionales, künstliches Zellgewebe entstehen zu lassen, wobei das Gesamtgemisch aus Trägersubstanzlösung, Zellsuspension und Nährlösung mindestens 5 Vol.% Matrigel enthält.

6. Verfahren nach Anspruch 5, **dadurch gekennzeichnet, daß** das Gesamtgemisch aus Trägersubstanzlösung, Zellsuspension und Nährlösung zwischen 5 und 15 Vol.% Matrigel enthält.

7. Verfahren nach Anspruch 6, **dadurch gekennzeichnet, daß** das Gesamtgemisch aus Trägersubstanzlösung, Zellsuspension und Nährlösung 10 Vol.% Matrigel enthält.

8. Verfahren nach einem der Ansprüche 5 bis 7, **dadurch gekennzeichnet, daß** die Trägersubstanz Kollagen ist.

9. Verfahren nach einem der Ansprüche 5 bis 8, **dadurch gekennzeichnet, daß** die Kardiomyozyten Säugerkardiomyozyten sind.

## Claims

1. An artificially produced, three-dimensional mammalian heart muscle tissue, comprising a support substance and muscle cells, obtainable by
- producing a mixture of a support substance solution and a nutrient solution,
- neutralizing the mixture,
- adding of extra cellular matrix from the Engelbreth-Holm-Swarm tumor, known as Matrigel^{™}, and of a muscle cell suspension to the mixture, whereby the whole mixture comprising support substance solution, cell suspension, and nutrient solution, contains at least 5 % by volume of Matrigel,
- incubating the mixture under conditions such that an artificial, three-dimensional muscle tissue is produced, and
- prestreching the formed muscle tissue from 3 to 20 % of the initial length.

2. The artificially produced, three-dimensional mammalian heart muscle tissue according to claim 1, **characterized in that** the formed muscle tissue is prestreched for 10 % of the initial length.

3. The artificially produced, three-dimensional mammalian heart muscle tissue according to claim 1 or 2, **characterized in that** the prestreching is carried out for a period of 1 to 7 days.

4. The artificially produced, three-dimensional mammalian heart muscle tissue according to one of claims 1 to 3, **characterized in that** the whole mixture comprising support substance solution, cell suspension, and nutrient solution contains between 5 and 15 % by volume of Matrigel.

5. A method for cultivating a cell culture in a support substance, whereby a support substance, a cell suspension, and a nutrient solution comprising the extra cellular matrix from the Engelbreth-Holm-Swarm tumor (Matrigel) are combined, **characterized in that** the cell suspension contains cardiomyocytes, and the whole mixture comprising support substance solution, cell suspension, and nutrient solution has a Matrigel concentration, which is sufficient for allowing a three-dimensional, artificial cell tissue to develope, whereby the whole mixture comprising support substance, cell suspension, and nutrient solution contains at least 5 % by volume of Matrigel.

6. The method according to claim 5, **characterized in that** the whole mixture comprising support substance solution, cell suspension, and nutrient solution contains between 5 and 15 % by volume of Matrigel.

7. The method according to claim 6, **characterized in that** the whole mixture comprising support substance solution, cell suspension, and nutrient solution contains 10 % by volume of Matrigel.

8. The method according to one of claims 5 to 7, **characterized in that** the support substance is collagen.

9. The method according to one of claims 5 to 8, **characterized in that** the cardiomyocytes are mammalian cardiomyocytes.

## Revendications

1. Tissu de myocarde de mammifère en trois dimensions, fabriqué par voie de synthèse, contenant une substance de support et des cellules musculaires, obtenu par :
- préparation d'un mélange d'une solution de substance de support et d'une solution de substance nutritive,
- neutralisation du mélange,
- ajout d'une matrice extracellulaire issue de la tumeur de Engelbreth-Holm-Swarm et connue en tant que Matrigel^{™}, et d'une suspension de cellules musculaires au mélange, suite à quoi le mélange total composé de la substance de solution de support, de la suspension cellulaire et de la solution nutritive contient au moins 5 % en volume de Matrigel,
- incubation du mélange dans des conditions telles qu'il se forme un tissu musculaire artificiel en trois dimensions, et
- pré-extension du tissu musculaire formé de 3 à 20 % de la longueur de départ.

2. Tissu de myocarde de mammifère en trois dimensions, fabriqué par voie de synthèse, selon la revendication 1, **caractérisé en ce que** le tissu musculaire formé est pré-étendu de 10 % de sa longueur de départ.

3. Tissu de myocarde de mammifère en trois dimensions, fabriqué par voie de synthèse, selon la revendication 1 ou 2, **caractérisé en ce que** la pré-extension est conduite sur une durée de 1 à 7 jours.

4. Tissu de myocarde de mammifère en trois dimensions, fabriqué par voie de synthèse, selon l'une des revendications 1 à 3, **caractérisé en ce que** le mélange total composé de la substance de solution de support, de la suspension cellulaire et de la solution nutritive contient 5 % à 15 % en volume de Matrigel.

5. Procédé de culture d'une culture cellulaire dans une substance de support, dans lequel on mélange une substance de support, une suspension cellulaire et une solution nutritive contenant la matrice extracellulaire de la tumeur de Engelbreth-Holm-Swarm (Matrigel), **caractérisé en ce que** la suspension cellulaire contient des cardiomyocytes et **en ce que** le mélange total de la solution de substance de support, de la suspension cellulaire et de la solution nutritive présente une concentration en Matrigel qui suffit pour créer un tissu cellulaire artificiel en trois dimensions, le mélange total de la solution de substance de support, de la suspension cellulaire et de la solution nutritive contenant au moins 5 % en volume de Matrigel.

6. Procédé selon la revendication 5, **caractérisé en ce que** le mélange total de la solution de substance de support, de la suspension cellulaire et de la solution nutritive contient 5 à 15 % en volume de Matrigel.

7. Procédé selon la revendication 6, **caractérisé en ce que** le mélange total de la solution de substance de support, de la suspension cellulaire et de la solution nutritive contient 10 % en volume de Matrigel.

8. Procédé selon l'une des revendications 5 à 7, **caractérisé en ce que** la substance de support est du collagène.

9. Procédé selon l'une des revendications 5 à 8, **caractérisé en ce que** les cardiomyocytes sont des cardiomyocytes de mammifères.
